(19) Europäisches Patentamt
European Patent Office
Office européen des brevets

(11) **EP 1 435 898 B1**

(12) **FASCICULE DE BREVET EUROPEEN**

(45) Date de publication et mention
de la délivrance du brevet:
**29.12.2010 Bulletin 2010/52**

(21) Numéro de dépôt: **02800623.7**

(22) Date de dépôt: **02.10.2002**

(51) Int Cl.:
*A61Q 1/02* (2006.01)    *A61K 8/34* (2006.01)
*A61K 8/55* (2006.01)    *A61K 8/60* (2006.01)

(86) Numéro de dépôt international:
**PCT/FR2002/003352**

(87) Numéro de publication internationale:
**WO 2003/030838 (17.04.2003 Gazette 2003/16)**

(54) **COMPOSITIONS EMULSIONNANTES A BASE D'ALCOOL GRAS ET D'ALKYLPOLYGLYCOSIDES**

EMULGIERENDE ZUSAMMENSETZUNG AUS FETTALKOHOL UND ALKYLPOLYGLYCOSIDEN

EMULSIFYING COMPOSITIONS BASED ON FATTY ALCOHOL AND ALKYLPOLYGLYCOSIDES

(84) Etats contractants désignés:
**AT BE BG CH CY CZ DE DK EE ES FI FR GB GR IE IT LI LU MC NL PT SE SK TR**

(30) Priorité: **11.10.2001 FR 0113088**

(43) Date de publication de la demande:
**14.07.2004 Bulletin 2004/29**

(60) Demande divisionnaire:
**10192510.5**

(73) Titulaire: **SOCIETE D'EXPLOITATION DE PRODUITS POUR LES INDUSTRIES CHIMIQUES, S.E.P.P.I.C.**
**75321 Paris Cédex 07 (FR)**

(72) Inventeurs:
• **ROSO, Alicia**
**Longuegineste, 81710 SAIX (FR)**
• **AMALRIC, Chantal**
**F-81700 Blan (FR)**
• **MICHEL, Nelly**
**94700 MAISONS ALFORT (FR)**
• **BOITEUX, Jean-Pierre**
**F-81710 Saix (FR)**
• **TABACCHI, Guy**
**F-75007 Paris (FR)**

• **MILIUS, Alain**
**F-06000 Nice (FR)**

(74) Mandataire: **Conan, Philippe Claude et al**
**L'Air Liquide**
**D.S.P.I.**
**75, quai d'Orsay**
**75321 Paris Cedex 07 (FR)**

(56) Documents cités:
WO-A-01/41896    WO-A-97/46219
WO-A-99/13830    WO-A-99/59537
FR-A- 2 756 195    FR-A- 2 762 317
US-A- 5 868 826

• PATENT ABSTRACTS OF JAPAN vol. 011, no. 379 (C-463), 10 décembre 1987 (1987-12-10) & JP 62 148419 A (LION CORP), 2 juillet 1987 (1987-07-02)
• PATENT ABSTRACTS OF JAPAN vol. 016, no. 071 (C-0913), 21 février 1992 (1992-02-21) & JP 03 264514 A (LION CORP), 25 novembre 1991 (1991-11-25)
• D. MILLER ET AL : "O/W emulsions for cosmetic products stabilized by alkyl phosphates - rheology and storage tests" COLLOIDS AND SURFACES. A, PHYSICACHEMICAL AND ENGINEERING ASPECTS, vol. 152, no. 1-2, 1999, pages 155-160, XP002230451 ELSEVIER, AMSTERDAM., NL ISSN: 0927-7757

**EP 1 435 898 B1**

**Description**

La présente invention a pour objet des compositions émulsionnantes particulières trouvant notamment application dans le domaine cosmétique.

Les esters phosphoriques d'alcools gras notamment les alkylphosphates préparés à partir d'un alcool gras ayant de 14 à 18 atomes de carbone sont connus comme étant des émulsionnants. L'utilisation des cétylphosphates comme émulsionnants en cosmétique est rapportée par POLO et al. dans DCI (sept 1999) 26-34 et 82-84.

Miller et al. dans Colloids and Surface A : Physiochemical and Engineering Aspects 152 (1999) 155-160 rapporte la préparation d'émulsions stables appropriées pour la cosmétique et basées sur des mélanges d'alcools gras et de leurs acides phosphoriques correspondant.

Les formulateurs cosmétiques recherchent en permanence de nouveaux touchers d'émulsions ne présentant pas l'inconvénient d'être riche. La demande de brevet FR-A-2 762 317 décrit notamment des associations alkylpolyglycosides C20-22 + alcools C20-22 conduisant à des émulsions peu grasses et évanescentes mais qui sont encore jugées trop grasses pour certains types de peaux. Un des problèmes à résoudre consiste donc à disposer d'émulsions qui présentent un toucher cosmétique supérieur à celui des émulsions traditionnelles en alliant pénétration rapide, toucher léger, non gras et sensation de fraîcheur.

Les émulsions à phase continue aqueuse sont connues pour leur faible résistance à l'eau et le formulateur essaye de pallier à ce défaut en combinant à son système émulsionnant des additifs tels que des polymères hydrophobes (ANTARON, GANEX, DERMACRYL LT79,GLOSSAMER) et/ou des co-émulsionnants notamment des cétylphosphates comme ceux de la gamme AMPHISOL. Ces additifs laissent sur la peau un film résiduel important. Un autre problème consiste à disposer d'émulsions qui présentent une meilleure résistance à l'eau combinée à l'absence de sensation de résidu.

Les émulsions de maquillage (fond de teint, mascaras, ombre à paupière crème) à phase aqueuse continue sont majoritairement formulées à base d'acide stéarique ou de dérivé d'acide stéarique (stéarate de PEG, stéarate de glycéryle). La stabilisation des charges minérales dans ces émulsions est un point critique : la tendance est d'obtenir une réagglomération des pigments au cours du temps qui compromet la texture lisse et uniforme (grains à l'application ou bien migration des pigments au sein du produit avec apparition de stries colorées). Les systèmes stabilisants de la phase aqueuse, associés au système émulsionnant, sont donc sophistiqués et incluent la plupart du temps la présence d'au moins deux polymères (d'origine synthétique ou naturelle). Par ailleurs ces émulsions à phase continue aqueuse ont l'inconvénient de présenter sur la peau des phénomènes de migration importants au cours de la journée. Enfin du fait de la nature et de la performance des émulsionnants utilisés, ces émulsions à phase continue aqueuse se trouvent généralement formulées à un pH voisin de 7 soit un pH nettement plus élevé que le pH cutané moyen (5,5).

Un autre problème consiste à disposer d'émulsions qui présentent une meilleure tenue du maquillage.

Un autre problème consiste à formuler des émulsions dans lesquelles les charges et/ou pigments éventuellement présents sont stabilisés de manière facile et durable avec un ajout minimal de stabilisants dans la phase aqueuse.

Un autre problème consiste à formuler des émulsions de maquillage contenant des pigments, notamment du dioxyde de titane, à un pH voisin du pH cutané moyen, en particulier compris entre 5 et 6.

Il a maintenant été découvert de façon inattendue, et c'est le fondement de l'invention, que l'association d'un alcool ayant 20 ou 22 atomes de carbone et d'un alcool phosphaté ayant 20 ou 22 atomes de carbone permet (i) d'obtenir des émulsions à toucher amélioré, (ii) d'améliorer la résistance à l'eau des émulsions, et (iii) d'améliorer les émulsions de maquillage.

Ainsi, selon un premier aspect, l'invention a pour objet une composition émulsionnante comprenant

- 10 à 90 % en poids, de préférence 15 à 90 % en poids, et de préférence encore 30 à 70 % en poids, d'alcool arachidylique et/ou d'alcool béhénylique ; et
- 90 à 10 % en poids, de préférence 85 à 10 % en poids, et de préférence encore 70 à 30 % en poids, d'ester phosphorique de l'alcool arachidylique et/ou d'ester phosphorique de l'alcool béhénylique (ci-après également désigné par "alcool(s) phosphaté(s)").

Par "ester phosphorique de l'alcool arachidylique et/ou béhénylique", on entend un monoester, un diester, un triester, ou un mélange en toutes proportions de mono-, di- et triester, de l'alcool concerné.

Selon un mode de réalisation particulier, le mélange d'alcool(s) et d'alcool(s) phosphaté(s) peut comprendre en outre jusqu'à 20 % en poids, de préférence de 1 à 10 % en poids, d'un ou plusieurs alkylpolyglycosides de formule $R\text{-}O\text{-}(Y)_p$ dans laquelle R représente un radical alkyle ayant de 14 à 22 atomes de carbone, de préférence de 20 à 22 atomes de carbone, Y représente le reste d'un ose en C5 ou C6, de préférence d'un glucose ou d'un xylose, et p représente un nombre décimal compris dans la gamme de 1 à 5, de préférence dans la gamme de 1 à 2,5 et de manière particulièrement préférée dans la gamme de 1 à 2.

Dans la formule $R\text{-}O\text{-}(Y)_p$, le groupe R-O- est lié à Y par le carbone anomérique du reste glucose ou xylose, de manière à former une fonction acétal.

Lorsque la composition émulsionnante conforme à l'invention comprend un ou plusieurs alkylpolyglycosides, elle peut être préparée soit de manière conventionnelle par mélange des différents constituants, soit de manière plus originale par un procédé qui fait intervenir une réaction *"one pot"*.

Ainsi, selon un second aspect, l'invention a pour objet un procédé de préparation d'une telle composition émulsionnante qui comprend :

(i) la phosphatation de l'alcool ou des alcools ; et
(ii) la réaction de l'alcool ou des alcools n'ayant pas réagi avec un sucre réducteur (ose en C5 ou C6) tel que le glucose ou le xylose.

La seconde étape du procédé est généralement effectuée en présence de catalyseurs acides forts comme par exemple l'acide sulfurique.

Ce procédé est très facile à mettre en oeuvre puisque les deux étapes décrites ci-dessus sont effectuées dans la même unité industrielle.

La composition conforme à l'invention permet de formuler très facilement des émulsions, notamment des émulsions de maquillage et des émulsions solaires.

Ainsi, selon un troisième aspect, l'invention a pour objet l'utilisation de la composition décrite ci-dessus comme agent émulsionnant pour la préparation d'émulsions.

Selon un quatrième aspect, l'invention a également pour objet une émulsion contenant la composition émulsionnante décrite ci-dessus. Cette composition représente généralement de 0,2 à 10 % en poids de l'émulsion.

L'émulsion comprend également de 1 à 50 % en poids, de préférence de 5 à 35 % en poids, et de préférence encore de 5 à 25 % en poids, d'une phase grasse constituée d'une ou plusieurs huiles et/ou d'une ou plusieurs cires.

L'huile est avantageusement choisie parmi les huiles suivantes :

- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales et leurs esters méthyliques éthoxylés ;
- les huiles d'origine animale, telles que le squalène, le squalane ;
- les huiles minérales, telles que l'huile de paraffine, l'huile de vaseline, et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone.

Cette huile peut également être choisie parmi les acides gras, les alcools gras, les cires d'origine naturelle ou synthétique, et plus généralement encore tout corps gras d'origine végétale, animale ou synthétique.

La cire est avantageusement choisie parmi les corps gras solides à température ambiante comme par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

L'émulsion conforme à l'invention peut également comprendre jusqu'à 10 % en poids d'un agent stabilisant comme par exemple le silicate de magnésium ; le silicate d'aluminium ; le silicate de sodium ; la gomme xanthane ; la gomme d'acacia ; la gomme de caroube ; la gomme de scléroglucane ; la gomme de gellane ; les alginates ; la cellulose et les dérivés de cellulose ; les argiles ; les amidons et les dérivés d'amidon ; les polymères et copolymères d'acide acrylique ; les polymères et copolymères d'acryloyldiméthyltaurate ; la polyvinylpyrrolidone ; les polymères et copolymères d'acrylamide ; les polyuréthanes.

De manière générale, l'émulsion conforme à l'invention peut également contenir jusqu'à 10 % en poids d'un ou plusieurs additifs habituellement utilisés en cosmétique et choisis parmi :

- les co-émulsionnants comme par exemple les acides gras ; les acides gras éthoxylés ; les esters d'acide gras et de sorbitol ; les esters d'acides gras éthoxylés ; les polysorbates ; les esters de polyglycérol ; les alcools gras éthoxylés ; les esters de sucrose ; les alkylpolyglycosides ; les alcools gras sulfatés et phosphatés ;
- les dispersants usuellement utilisés en cosmétique notamment le laurylsulfate ; le lauryléthersulfate ; l'octénylsulfosuccinate ; le laurylsarcosinate ; le diméthicone-copolyolphosphate comme par exemple le produit commercialisé sous la dénomination PECOSIL® PS100 ; les polymères et copolymères acryliques comme par exemple ceux commercialisés sous la dénomination AVALURE® ; les lauroylaminoacides comme par exemple le produit commercialisé sous la dénomination PROTEOL OAT® ;
- les conservateurs habituellement utilisés en cosmétique ;
- les parfums ou autres additifs à fonction parfumante (comme notamment les huiles essentielles et les cires essentielles) ;
- le DHA ; les actifs antirides, hydratants, apaisants, anti-radicalaires, antioxydants, séborégulateurs ; les sels minéraux ; les vitamines ; les phytostérols ; les polyphénols ; les sphingolipides.

Selon un mode de réalisation avantageux, l'émulsion conforme à l'invention est une émulsion de maquillage. Dans ce cas de figure particulier, l'émulsion comprend de 0,5 à 50 % en poids, de préférence de 2 à 35 % en poids, de pigments et/ou charges organiques ou inorganiques.

Ces pigments et/ou charges peuvent être lamellaires ou sphériques et sans limitation particulière quant à leur granulométrie. A titre d'exemple on peut citer notamment le poly(méthacrylate de méthyle) ; les copolymères réticulés d'acide méthacrylique ; le nylon ; la poly(bêta-alanine) ; le polyéthylène ; le téflon ; la lauroyllysine ; les poudres d'alkylaminoacide, d'amidon, de PTFE ; les microsphères creuses comme par exemple celles commercialisées sous la dénomination EXPANCELL® ou POLYTRAP® ; les microbilles de résine de silicone ; le dioxyde de titane ; l'oxyde de zinc ; l'oxyde de fer (noir - rouge - jaune) ; le titanate de fer ; le noir de carbone ; l'oxyde de chrome ; l'hydroxyde de chrome ; l'oxyde de zirconium ; l'oxyde de cérium ; le titanate de cobalt ; l'ultramarine ; le bleu de Prusse ; le micatitane ; l'oxychlorure de bismuth ; l'essence d'orient ; le talc ; la poudre d'aluminium ; la poudre de cuivre ; la poudre d'or ; le mica ; la séricite ; le kaolin ; la phlogopite ; le mica synthétique ; la lépidolite ; la biotite ; la vermiculite ; le carbonate de calcium ; le carbonate et l'hydroxycarbonate de magnésium ; le silicate d'aluminium ; le silicate de baryum ; le silicate de calcium ; le silicate de magnésium ; le silicate de strontium ; la silice et les microsphères de silice ; les microsphères de céramique et de verre ; les zéolites ; l'hydroxyapatite, les sels métalliques de l'acide tungstique ; le sulfate de baryum ; le gypse ; le phosphate de calcium ; les savons métalliques d'acides gras ; le nitrure de bore ; les pigments photochromiques ; les pigments interférentiels. Ces charges peuvent avoir subi un traitement de surface.

L'émulsion de maquillage selon la présente invention peut également comprendre jusqu'à 20 % en poids, de préférence jusqu'à 10 % en poids, d'un ou plusieurs co-solvants comme par exemple le glycérol ; le sorbitol ; le PEG ; le monopropylèneglycol ; le butylèneglycol ; l'isoprèneglycol ; le méthyl-2-propanediol-1,3 ; l'éthanol ; l'hexylèneglycol.

Selon un autre mode de réalisation avantageux, l'émulsion conforme à l'invention est une émulsion solaire. Dans ce cas de figure particulier, l'émulsion comprend de 1 à 40 % en poids, de préférence de 1 à 20 % en poids, d'un ou plusieurs filtres ou écrans solaires organiques ou inorganiques ; et jusqu'à 30 % en poids, de préférence jusqu'à 10 % en poids, d'un agent résistant à l'eau.

L'émulsion conforme à l'invention peut également contenir une quantité suffisante de base comme par exemple la soude, la potasse, l'ammoniaque, la triéthanolamine, la tétrahydroxypropyléthylènediamine, le trishydroxyami-nométhane, l'aminométhylpropanol, pour ajuster le pH final de l'émulsion entre 3 et 10.

Bien évidemment, la somme des différents constituants de l'émulsion selon l'invention est égale à 100 %.

Les émulsions conformes à l'invention peuvent être préparées selon le mode opératoire décrit ci-dessous.

La phase aqueuse contenant éventuellement le(s) co-solvant(s) et la base, est chauffée à une température de 70 à 85°C. Cette phase aqueuse contient le cas échéant les charges et/ou les pigments, qui peuvent être préalablement broyés ; les agents stabilisants ; les dispersants ; ainsi que les conservateurs, parfums et autres actifs.

Parallèlement la phase grasse contenant la composition émulsionnante, les huiles et/ou les cires, est chauffée à une température identique de 70 à 85°C.

Les deux phases sont ensuite mélangées et émulsionnées à l'aide d'un appareil de type rotor-stator (mélangeur de laboratoire SILVERSON ou appareils industriels OLSA - RAYNERI - BECOMIX). Après quelques minutes d'émulsification, l'émulsion est refroidie sous agitation modérée.

L'invention est illustrée à l'aide des exemples suivants.

**EXEMPLE 1 : Synthèse d'un émulsionnant selon l'invention**

50 kg d'alcool (mélange d'alcools en C20 et C22 dans un ratio 70/30), sont introduits dans un réacteur pré-chauffé à 70°C, sous un léger barbotage d'azote. Une fois le mélange d'alcools fondu, 3,88 kg d'anhydride phosphorique sont ajoutés progressivement. Le milieu réactionnel est maintenu 4h30 à 85°C, puis vidangé.

Le produit obtenu possède les caractéristiques suivantes :

- aspect solide blanc
- indice d'acide 90,4 mg KOH/g
- alcool C20-C22 46,0 %
- alcool C20-C22 phosphaté 54,0 %.

**EXEMPLE 2 : Synthèse d'un émulsionnant selon l'invention**

24,3 kg du produit obtenu selon l'exemple 1 sont mis à réagir à 110°C avec 1,13 kg de glucose anhydre en présence catalytique d'acide sulfurique pendant 8 heures. Le produit obtenu après filtration présente les caractéristiques suivantes :

- aspect solide blanc
- alcool C20-C22 43 %
- alcool C20-C22 phosphaté 54 %
- alkypolyglucoside C20-C22 3 %

Pour mettre en évidence l'invention les émulsionnants testés sont indiqués dans le Tableau 1. Les émulsionnants C et G sont des émulsionnants selon l'invention. Les autres sont des comparatifs.

TABLEAU 1

| | | EMULSIONNANT |
|---|---|---|
| | A | Alcool C16 phosphaté + alcool C16-18 (50/50) |
| | B | Alcool C16 phosphaté + alcool C20-22 (50/50) |
| | C (ex 1) | Alcool C20-22 phosphaté + alcool C20-22 (54/46) |
| | D | Alkylpolyglucoside C20-22 + alcool C20-22 (20/80) |
| | E | Alcool C20-22 phosphaté |
| | F | Alcool C20-22 phosphaté + alcool C16-18 (50/50) |
| | G (ex 2) | Alcool C20-22 phosphaté + alcool C20-22 + alkylpolyglucoside C-20-22 (54/43/3) |
| | H | Acide stéarique + stéarate de PEG 100 (67/33) |

L'alcool C16 phosphaté est commercialisé par la Société GIVAUDAN-ROURE sous la dénomination Amphisol® A.
Les alcools C20-22 et les alcools C16-18 sont respectivement des mélanges 70/30 et 50/50.
L'alcool C20-22 phosphaté est préparé dans les conditions de l'exemple 1 en utilisant 0,75 kg d'alcool en C20-22 et 0,22 kg d'anhydride phosphorique.

**EXEMPLE 3 : Mise en évidence de l'amélioration du toucher**

On prépare des émulsions ayant la composition suivante.

| Phase grasse | |
|---|---|
| Emulsionnant | 5% |
| Octanoate de cétéaryle | 15% |

| Phase aqueuse | |
|---|---|
| Eau | qsp 100% |

(suite)

| Phase aqueuse | |
|---|---|
| Trométhamine | qs pH=5,5 |
| Conservateurs | qs |

Les émulsions préparées sont contrôlées par une évaluation sensorielle : les critères de confort sont notés de 0 à 5 par un jury d'experts après application sur le visage. Sont évaluées : la sensation de gras (0 = non gras et 5 = très gras) et la présence de résidu (0 = absence de résidu et 5 = résidu important).

Les résultats sont présentés dans le Tableau 2.

TABLEAU 2

| émulsionnant | A | B | C | D | E | F |
|---|---|---|---|---|---|---|
| évaluation sensorielle : | | | | | | |
| - gras | 3 | 3 | 0 | 2 | - | 3 |
| - résidu | 4 | 2 | 0 | 1 | - | 4 |

L'émulsionnant selon l'invention se distingue par son toucher non gras ainsi que par l'absence de résidu sur la peau. A noter que l'émulsion E n'est pas stable.

**EXEMPLE 4 : Mise en évidence de l'amélioration de la résistance à l'eau**

On prépare des émulsions ayant la composition suivante :

| | | | |
|---|---|---|---|
| A | Emulsionnant | 3 % |
| | Adipate de diisopropyle | 10 % |
| | Glycérine | 7 % |
| | Méthoxycinnamate d'éthylhexyle | 7,50 % |
| | Octocrylène | 10 % |
| | Butylméthoxydibenzoylméthane | 2 % |
| | | |
| B | Copolymère acrylamide/acryloyldiméthyltaurate et isohexadécane et polysorbate 80 | 1,20 % |
| | Cyclométhicone | 5 % |
| | | |
| C | EDTA tétrasodique | 0,20 % |
| | Gomme xanthane | 0,15 % |
| | Silicate de magnésium et d'aluminium | 0,15 % |
| | Eau | qsp 100 % |
| | | |
| D | Conservateur | qs |
| | Trométhamine | qs pH = 5 |

La résistance à l'eau est déterminée *in vivo* sur un panel de volontaires selon le protocole suivant :

Phase 1

Détermination sur le dos de zones cutanées : une zone par émulsion testée + une zone témoin sur laquelle aucune émulsion n'est appliquée.

Application des émulsions à raison de 2 mg/cm$^2$.

Irradiation UV sur l'ensemble des zones 15 minutes après l'application des émulsions (lampe xenon).

16 à 24 heures après l'irradiation, lecture visuelle des doses érythémales minimales sur l'ensemble des zones (DEM)

Calcul des indices de protection solaire des produits testés :

$$IP\ produit = \frac{DEM\ zone\ produit}{DEM\ zone\ témoin}$$

Phase 2

Détermination de nouvelles zones cutanées sur le dos : une zone par émulsion testée et une zone témoin.

Application des émulsions à raison de 2 mg/cm$^2$.

Réalisation de deux bains de 20 minutes chacun espacés de 10 minutes. Irradiation UV sur l'ensemble des zones 15 minutes après les bains (lampe xenon).

Lecture visuelle des doses érythémales minimales sur l'ensemble des zones après bain.

Calcul de l'indice de protection solaire après bain

$$IP\ produit = \frac{DEM\ zone\ produit\ après\ bain}{DEM\ zone\ témoin\ après\ bain}$$

$$résistance\ à\ l'eau = \frac{indice\ de\ protection\ solaire\ après\ bain\ (phase\ 2)}{indice\ de\ protection\ solaire\ (phase\ 1)}$$

Les émulsions préparées sont contrôlées par une évaluation sensorielle.

Les résultats sont présentés dans le Tableau 3.

TABLEAU 3

| Emulsionnant | Résistance à l'eau % | Evaluation sensorielle : gras | Evaluation sensorielle : résidu |
|---|---|---|---|
| A | 18 | 3 | 4 |
| B | 17 | 3 | 2 |
| C | 35 | 0 | 0 |
| G | 41 | 0 | 0 |

Les émulsions selon l'invention présente une résistance à l'eau significativement améliorée sans toutefois laisser sur la peau de sensation grasse ou de résidu.

**EXEMPLE 5 : Mise en évidence de l'amélioration des émulsions de fond de teint**

On prépare des émulsions ayant la composition suivante :

Phase grasse

| | |
|---|---|
| Emulsionnant | 3 % |
| Isononanoate d'isononyle | 10 % |
| Citrate de triisostéaryle | 10 % |

Phase aqueuse

| | |
|---|---|
| Eau | qsp 100 % |
| Oxydes de fer | 1,2 % |

(suite)

| Phase aqueuse | |
|---|---|
| Diméthicone PEG 7 phosphate | 0,8 % |
| Oxyde de titane | 12 % |
| Talc | 4% |
| Butylèneglycol | 4 % |
| PEG 400 | 4% |
| Trishydroxyaminométhane | qs pH 7 |
| Polyacrylamide et C13-C14 isoparraffine et Laureth-7 | 1,5% |

Les émulsions sont contrôlées :

* par un suivi visuel (macroscopique) de la stabilité des émulsions avec vérification après 3 mois de l'aspect des émulsions dans le flacon : aspect lisse ou granuleux, aspect brillant ou mat, suivi des phénomènes de déphasage, de relargage de pigments en surface de l'émulsion ou bien de stratification des pigments avec un effet visuel hétérogène. Les critères optimaux sont une émulsion brillante, parfaitement lisse et homogène sans déphasage ni relargage ou stratification des pigments et charges. La notation est la suivante : + si tous les critères sont satisfaisants, +/- si un des critères au moins est non satisfaisant, 0 si aucun des critères n'est satisfaisant.
* par un suivi de la texture avec réalisation sur plaque plexiglas de films calibrés à 120 $\mu$m et vérification de l'absence d'agglomérats de charges et pigments. La notation est la suivante : + en l'absence de grains 3 mois après la fabrication de l'émulsion, +/- en présence de quelques grains, 0 en présence de nombreux grains.
* par une évaluation de la tenue de l'émulsion sur le visage par un maquilleur professionnel 4 h après l'application. La notation est la suivante : + en l'absence de migration des pigments et charges dans les rides et ridules, +/- en présence d'une migration modérée, 0 en présence d'une migration importante.
* par une évaluation sensorielle : les critères de confort sont notés de 0 à 5 par un jury d'experts après application sur le visage. Sont évaluées : la sensation de gras (0 = non gras et 5 = très gras), la sensation de fraîcheur (0 = absence de fraîcheur et 5 = fraîcheur importante), la facilité d'étalement (0 = difficile à étaler et 5 = facile à étaler).

Les résultats sont présentés dans le Tableau 4

TABLEAU 4

| Emulsionnant | A | C | D | G | H |
|---|---|---|---|---|---|
| contrôle visuel | + | + | + | + | +/- |
| suivi texture | +/- | + | +/- | + | +/- |
| migration | +/- | + | 0 | + | 0 |
| évaluation sensorielle : - gras - fraîcheur - facilité d'étalement | 3 3 3 | 2 4 4 | 2 4 5 | 2 4 5 | 2 3 3 |

Parmi les émulsionnants testés, ceux de l'invention apportent simultanément un maximum de critères recherchés.

**EXEMPLE 6 : Mise en évidence de l'amélioration des émulsions de fond de teint à pH acide**

On prépare des émulsions à pH 5 ayant la composition indiquée à l'exemple 5 avec les émulsionnants A, G, H.
Les résultats sont présentés dans le Tableau 5.

TABLEAU 5

| Emulsionnant | A | G | H |
|---|---|---|---|
| contrôle visuel | +/- | + | 0 |

(suite)

| Emulsionnant | A | G | H |
|---|---|---|---|
| suivi texture | 0 | +/- | 0 |

Parmi les émulsionnants testés, celui de l'invention apporte la plus grande souplesse dans l'ajustement du pH en milieu acide. Pour les autres émulsionnants, l'acidification du pH apporte une instabilité rédhibitoire des pigments et charges.

**Revendications**

1. Composition émulsionnante, **caractérisée en ce qu'**elle comprend :

   - 10 à 90 % en poids, de préférence 15 à 90 % en poids, de préférence encore 30 à 70 % en poids, d'alcool arachidylique et/ou d'alcool béhénylique ;
   - 90 à 10 % en poids, de préférence 85 à 10 % en poids, de préférence encore 70 à 30 % en poids, d'ester phosphorique de l'alcool arachidylique et/ou d'ester phosphorique de l'alcool béhénylique ;
   - jusqu'à 20 % en poids, de préférence de 1 à 10 % en poids, d'un ou plusieurs alkylpolyglycosides de formule $R-O-(Y)_p$ dans laquelle :
   - R représente un radical alkyle ayant de 14 à 22 atomes de carbone, de préférence de 20 à 22 atomes de carbone,
   - Y représente le reste d'un ose en C5 ou en C6, de préférence d'un glucose ou d'un xylose, et
   - p représente un nombre décimal compris dans la gamme de 1 à 5, de préférence dans la gamme de 1 à 2,5 et de manière particulièrement préférée dans la gamme de 1 à 2.

2. Composition selon la revendication 1, qui comprend de 1 à 20 % en poids, de préférence de 1 à 10 % en poids, d'alkylpolyglycoside(s).

3. Procédé de préparation de la composition selon la revendication 2, **caractérisé en ce qu'**il comprend :

   - la phosphatation de l'alcool arachidylique et/ou de l'alcool béhénylique ;
   - la réaction de l'alcool (des alcools) n'ayant pas réagi avec l'ose en C5 ou en C6.

4. Utilisation de la composition selon la revendication 1 ou 2 comme agent émulsionnant pour la préparation d'émulsions.

5. Emulsion, **caractérisée en ce qu'**elle contient une composition selon la revendication 1 ou 2, ou une composition obtenue selon le procédé de la revendication 3.

6. Emulsion selon la revendication 5, **caractérisée en ce qu'**elle comprend :

   - de 0,2 à 10 % en poids d'une composition selon la revendication 1 ou 2 ;
   - de 0 à 10 % en poids d'un co-émulsionnant ;
   - de 1 à 50 % en poids, de préférence de 5 à 35 % en poids, et de préférence encore de 5 à 25 % en poids, d'une phase grasse constituée d'une ou plusieurs huiles et/ou d'une ou plusieurs cires ; et
   - de 0 à 10 % en poids d'un agent stabilisant.

7. Emulsion selon la revendication 5 ou 6, qui est une émulsion de maquillage.

8. Emulsion selon la revendication 7, **caractérisée en ce qu'**elle comprend en outre :

   - de 0 à 20 % en poids, de préférence de 0 à 10 % en poids, d'un ou plusieurs co-solvants ; et
   - de 0,5 à 50 % en poids, de préférence de 2 à 35 % en poids, de pigments et/ou charges organiques ou inorganiques.

9. Emulsion selon la revendication 5 ou 6, qui est une émulsion solaire.

10. Emulsion selon la revendication 9, **caractérisée en ce qu'**elle comprend :

    - de 1 à 40 % en poids, de préférence de 1 à 20 % en poids, d'un ou plusieurs filtres ou écrans solaires organiques ou inorganiques ; et
    - jusqu'à 30 % en poids, de préférence jusqu'à 10 % en poids, d'un agent résistant à l'eau.

11. Utilisation de la composition selon la revendication 1 ou 2 comme agent émulsionnant pour la préparation d'émulsions de maquillage contenant du dioxyde de titane à pH compris entre 5 et 6.

**Claims**

1. Emulsifying composition, **characterized in that** it comprises:

    - 10 to 90% by weight, preferably 15 to 90% by weight, more preferably 30 to 70% by weight, of arachidyl alcohol and/or of behenyl alcohol;
    - 90 to 10% by weight, preferably 85 to 10% by weight, more preferably 70 to 30% by weight, of phosphoric ester of arachidyl alcohol and/or of phosphoric ester of behenyl alcohol;
    - up to 20% by weight, preferably from 1 to 10% by weight, of one or more alkylpolyglycosides of formula R-O-(Y)$_p$, in which:
    - R represents an alkyl radical having from 14 to 22 carbon atoms, preferably from 20 to 22 carbon atoms,
    - Y represents the residue of a $C_5$ or $C_6$ monosaccharide, preferably of a glucose or of a xylose, and
    - p represents a decimal number within the range from 1 to 5, preferably within the range from 1 to 2.5 and particularly preferably within the range from 1 to 2.

2. Composition according to Claim 1, which comprises from 1 to 20% by weight, preferably from 1 to 10% by weight, of alkylpolyglycoside(s).

3. Process for the preparation of the composition according to Claim 2, **characterized in that** it comprises:

    - the phosphation of arachidyl alcohol and/or of behenyl alcohol;
    - the reaction of the unreacted alcohol (alcohols) with the $C_5$ or $C_6$ monosaccharide.

4. Use of the composition according to Claim 1 or 2 as emulsifying agent for the preparation of emulsions.

5. Emulsion, **characterized in that** it comprises a composition according to Claim 1 or 2 or a composition obtained according to the process of Claim 3.

6. Emulsion according to Claim 5, **characterized in that** it comprises:

    - from 0.2 to 10% by weight of a composition according to Claim 1 or 2;
    - from 0 to 10% by weight of a coemulsifier;
    - from 1 to 50% by weight, preferably from 5 to 35% by weight and more preferably from 5 to 25% by weight of a fatty phase composed of one or more oils and/or of one or more waxes; and
    - from 0 to 10% by weight of a stabilizing agent.

7. Emulsion according to Claim 5 or 6, which is a makeup emulsion.

8. Emulsion according to Claim 7, **characterized in that** it additionally comprises:

    - from 0 to 20% by weight, preferably from 0 to 10% by weight, of one or more cosolvents; and
    - from 0.5 to 50% by weight, preferably from 2 to 35% by weight, of organic or inorganic pigments and/or fillers.

9. Emulsion according to Claim 5 or 6, which is an antisun emulsion.

10. Emulsion according to Claim 9, **characterized in that** it comprises:

    - from 1 to 40% by weight, preferably from 1 to 20% by weight, of one or more organic or inorganic sunscreens; and

- up to 30% by weight, preferably up to 10% by weight, of a water-resisting agent.

**11.** Use of the composition according to Claim 1 or 2 as emulsifying agent for the preparation of makeup emulsions comprising titanium dioxide at a pH of between 5 and 6.


**Patentansprüche**

**1.** Emulgatorzusammensetzung, **dadurch gekennzeichnet, daß** sie folgendes umfaßt:

- 10 bis 90 Gew.-%, vorzugsweise 15 bis 90 Gew.-%, noch weiter bevorzut 30 bis 70 Gew.-%, Arachidylalkohol und/oder Behenylalkohol;
- 90 bis 10 Gew.-%, vorzugsweise 85 bis 10 Gew.-%, noch weiter bevorzugt 70 bis 30 Gew.-%, Phosphorsäureester von Arachidylalkohol und/oder Phosphorsäureester von Behenylalkohol;
- bis zu 20 Gew.-%, vorzugsweise 1 bis 10 Gew.-%, eines oder mehrerer Alkylpolyglykoside der Formel R-O-$(Y)_p$, worin:
- R für einen Alkylrest mit 14 bis 22 Kohlenstoffatomen, vorzugsweise 20 bis 22 Kohlenstoffatomen, steht,
- Y für den Rest einer $C_5$- oder $C_6$-Ose, vorzugsweise einer Glucose oder einer Xylose, steht und
- p für eine Dezimalzahl im Bereich von 1 bis 5, vorzugsweise im Bereich von 1 bis 2,5 und besonders bevorzugt im Bereich von 1 bis 2 steht.

**2.** Zusammensetzung nach Anspruch 1, die 1 bis 20 Gew.-%, vorzugsweise 1 bis 10 Gew.-%, Alkylpolyglykosid(e) umfaßt.

**3.** Verfahren zur Herstellung der Zusammensetzung nach Anspruch 2, **dadurch gekennzeichnet, daß** man:

- Arachidylalkohol und/oder Behenylalkohol phosphatiert;
- den nicht umgesetzten Alkohol bzw. die nicht umgesetzten Alkohole mit der $C_5$- oder $C_6$-Ose umsetzt.

**4.** Verwendung der Zusammensetzung nach Anspruch 1 oder 2 als Emulgator zur Herstellung von Emulsionen.

**5.** Emulsion, **dadurch gekennzeichnet, daß** sie eine Zusammensetzung nach Anspruch 1 oder 2 oder eine nach dem Verfahren gemäß Anspruch 3 erhaltene Zusammensetzung enthält.

**6.** Emulsion nach Anspruch 5, **dadurch gekennzeichnet, daß** sie folgendes umfaßt:

- 0,2 bis 10 Gew.-% einer Zusammensetzung nach Anspruch 1 oder 2;
- 0 bis 10 Gew.-% eines Coemulgators;
- 1 bis 50 Gew.-%, vorzugsweise 5 bis 35 Gew.-% und noch weiter bevorzugt 5 bis 25 Gew.-% einer Fettphase, die aus einem oder mehreren Ölen und/oder einem oder mehreren Wachsen besteht; und
- 0 bis 10 Gew.-% eines Stabilisators.

**7.** Emulsion nach Anspruch 5 oder 6, bei der es sich um eine Makeup-Emulsion handelt.

**8.** Emulsion nach Anspruch 7, **dadurch gekennzeichnet, daß** sie außerdem folgendes umfaßt:

- 0 bis 20 Gew.-%, vorzugsweise 0 bis 10 Gew.-%, eines oder mehrerer Cosolventien und
- 0,5 bis 50 Gew.-%, vorzugsweise 2 bis 35 Gew.-%, Pigmente und/oder organische oder anorganische Füllstoffe.

**9.** Emulsion nach Anspruch 5 oder 6, bei der es sich um eine Sonnenschutzemulsion handelt.

**10.** Emulsion nach Anspruch 9, **dadurch gekennzeichnet, daß** sie folgendes umfaßt:

- 1 bis 40 Gew.-%, vorzugsweise 1 bis 20 Gew.-%, eines oder mehrerer organischer oder anorganischer Lichtschutzmittel und
- bis zu 30 Gew.-%, vorzugsweise bis zu 10 Gew.-%, eines wasserfestmachenden Mittels.

11. Verwendung der Zusammensetzung nach Anspruch 1 oder 2 als Emulgator zur Herstellung von Titandioxid enthaltenden Makeup-Emulsionen bei einem pH-Wert zwischen 5 und 6.

## RÉFÉRENCES CITÉES DANS LA DESCRIPTION

**Documents brevets cités dans la description**

- FR 2762317 A

**Littérature non-brevet citée dans la description**

- **POLO et al.** *DCI,* Septembre 1999, 26-3482-84

- **Miller et al.** *Colloids and Surface A : Physiochemical and Engineering Aspects,* 1999, vol. 152, 155-160